# Europäisches Patentamt

# European Patent Office

# Office européen des brevets

(19)

(11) Publication number: **0 000 276**
**B1**

(12)

# EUROPEAN PATENT SPECIFICATION

(45) Date of publication of patent specification: **01.09.82**

(21) Application number: **78300082.1**

(22) Date of filing: **27.06.78**

(51) Int. Cl.³: **C 07 D 263/56,**
**A 61 K 31/42**

(54) **A novel crystalline form of benoxaprofen, methods of preparation thereof and pharmaceutical formulations containing said novel form.**

(30) Priority: **28.06.77 GB 2690777**

(43) Date of publication of application:
**10.01.79 Bulletin 79/1**

(45) Publication of the grant of the patent:
**01.09.82 Bulletin 82/35**

(84) Designated Contracting States:
**DE NL SE**

(56) References cited:
**DE - A - 2 450 053**
**NL - A - 73 07018**

**JOURNAL OF MEDICINAL CHEMISTRY 18**
**(1975), 53—58.**

(73) Proprietor: **Lilly Industries Limited**
**Henrietta House Henrietta Place**
**London W1M 0ED (GB)**

(72) Inventor: **Sherlock, Roy**
**"Downs End" 113, Wodeland Avenue**
**Guildford Surrey (GB)**
Inventor: **Hicks, Terence Alan**
**Rydal Close Cove**
**Farnborough Hampshire (GB)**

(74) Representative: **Crowther, Terence Roger**
**European Patent Attorney**
**Erl Wood Manor**
**Windlesham Surrey GU20 6PH (GB)**

Courier Press, Leamington Spa, England.

# A novel crystalline form of benoxaprofen, methods of preparation thereof and pharmaceutical formulations containing said novel form

This invention relates to a novel polymorphic form of a pharmacologically active substance and to pharmaceutical formulations containing the novel polymorph.

The compound 2-(4-chlorophenyl)-α-methyl-5-benzoxazolylacetic acid, hereinafter referred to by its generic name "benoxaprofen" is described in United Kingdom Patent Specification No. 1,435,721 and in an article in the *Journal of Medicinal Chemistry,* 18, 53 (1975) as a potent antiinflammatory agent, and is presently undergoing clinical trial. Melting point determinations disclosed in these publications show the compound to have a melting point in the range of 188°C to 190°C.

Both the patent specification and the article are silent as regards polymorphic properties of benoxaprofen. However, the laboratory procedures described in those publications result in formation of the kinetically preferred polymorphic form, hereinafter referred to as "Form I", of benoxaprofen. However, this kinetically favoured polymorph is not the thermodynamically stable form, which is hereinafter referred to as "Form II", and therefore tends to undergo polymorphic transformation on storage. For instance, random cycling experiments carried out at temperatures between 4 and 37°C have shown 20% transformation of Form I to Form II after two years. When one considers that pharmaceutical formulations containing drugs of this type would generally be expected to have a shelf life of the order of five years, it can be readily appreciated that the metastable nature of Form I presents serious problems with respect to commercially viable compositions such as tablets, creams and suspensions. For a discussion of some of the problems associated with the utilisation of metastable forms of drugs in pharmaceutical formulations reference may be made to the review article in *Journal of Pharmaceutical Sciences, 58,* 8, 911 (1969).

An object of the present invention is to provide a polymorphic form of benoxaprofen which has sufficient stability to be useful in the production of pharmaceutical formulations having a satisfactory shelf life.

According to the present invention there is provided a polymorphic form of benoxaprofen, crystals of which have the following powder diffraction pattern using filtered copper-nickel radiation at $\lambda=1.5405$ Å.

| "d" in Å | $I/I_o$ |
|---|---|
| 11.77 | 10 |
| 8.06 | 10 |
| 7.07 | 70 |
| 5.67 | 100 |
| 5.30 | 10 |
| 5.06 | 20 |
| 4.79 | 10 |
| 4.41 | 50 |
| 4.17 | 80 |
| 3.93 | 05 |
| 3.65 | 30 |
| 3.56 | 90 |
| 3.24 | 60 |
| 3.09 | 40 |
| 3.03 | 15 |
| 2.97 | 15 |
| 2.81 | 05 |
| 2.75 | 05 |
| 2.66 | 05 |
| 2.57 | 05 |
| 2.37 | 10 |
| 2.29 | 05 |
| 2.15 | 05 |
| 2.04 | 15 |
| 1.98 | 20 |
| 1.91 | 05 |
| 1.78 | 02 |

Form II, characterised as above, can also be distinguished from Form I by its infra red spectrum. Using a Perkin Elmer 297 spectrophotometer with benoxaprofen homogeneously dispersed in a potassium bromide disc, the following differences can be observed:—

1. Form I exhibits a sharp, medium intensity band at 880 cm⁻¹ whereas Form II exhibits a similar band at 885 cm⁻¹.

2. In the region 1200—1330 cm⁻¹ both forms show a similar positioning of bands, but the intensities differ. In Form I the bands at 1220 and 1250 cm⁻¹ are considerably more intense than the others, whilst in Form II all bands are of similar intensity.

3. The strong band near 1700 cm⁻¹ is considerably sharper for Form I than for Form II.

Infra red analysis, which will be the usual method of assay of commercial material, is sensitive enough to detect as little as 10% by weight of Form I in batches of Form II material. Batches of Form II assayed by this spectral mode of analysis have proved to be quite satisfactory in pharmaceutical formulations such as tablets, capsules or suspensions, such formulations not deteriorating on storage.

Accordingly, in a second aspect of the invention there is provided Form II contaminated with less than 10% by weight of Form I.

According to a further aspect of the invention there is provided a pharmaceutical formulation comprising as an active material Form II associated with a pharmaceutically-acceptable carrier therefor.

The Form II polymorph in the above formulation should be pure as determined by the infra red assay technique described previously, i.e. it should contain less than 10% by weight of Form I.

Form I can be converted to Form II by heating in a fluid bed dryer at a temperature of approximately 115°C for upwards of 3 hours. Alternatively, Form II may be prepared by slow and controlled crystallisation from solutions of benoxaprofen in n-butyl acetate.

Form II crystals of benoxaprofen can also be obtained by thermal decomposition at temperatures in the range 90—160°C of the ammonium salt of benoxaprofen. According to one aspect of this procedure, benoxaprofen ammonium salt is isolated directly from the hydrolysis of 2-(4-chlorophenyl)-α-methyl-5-benzoxazolylacetonitrile as an insoluble precipitate. The precipitate is collected and dried at a temperature in the above range, during which drying period the ammonium salt decomposes to yield dry benoxaprofen Form II crystals. Drying is continued until the decomposition of the ammonium salt is substantially complete. The yield of Form II material is usually in the range 95—98 percent.

Alternatively, the ammonium salt can be suspended in a solvent boiling in the range 90—160°C and the resulting suspension or slurry heated, preferably by reflux; i.e. at the boiling point of the solvent, until the ammonium salt is substantially completely decomposed to ammonia and the free purified alkanoic acid. If the purified acid thus produced is substantially insoluble in the solvent used to slurry the ammonium salt (n-octane for example), benoxaprofen Form II will be obtained as from heating the salt in the absence of a solvent. If benoxaprofen is soluble in the solvent used to slurry the ammonium salt, (n-butyl acetate for example), a recrystallized product will be obtained. With either type of solvent benoxaprofen can be separated from the solvent by decantation or filtration. If benoxaprofen is soluble in the solvent employed, the solution is ordinarily concentrated and/or chilled to increase crystallisation and further crystals are obtained from the mother liquor.

The following non-limitative Examples will serve to illustrate the invention, benoxaprofen Form I being produced as follows:

The process described in Method D, page 55 from *Journal of Medicinal Chemistry, 18* (1975) was repeated exactly. The recrystallisation procedure adopted was conventional, i.e. the solution of benoxaprofen in ethanol was formed by warming on a steam bath and cooling of the thus-formed solution was effected using an icebath.

Infra red analysis and X-ray powder diffraction both showed that exclusive formation of Form I had occurred.

Example 1

41 kg of 2-(4-chlorophenyl)-α-methyl-5-benzoxazolylacetonitrile were hydrolyzed in 12N aqueous hydrochloric acid by being stirred at 80°C for about two hours. The reaction mixture was cooled to about 40°C and then poured slowly with vigorous stirring into cold water. The solid precipitate of 2-(4-chlorophenyl)-α-methyl-5-benzoxazolylacetic acid thus prepared was collected by filtration and the filter cake washed with water until the washings no longer gave an acidic reaction to litmus. The filter cake was dried at 70—80°C; yield=40 kg (77 percent purity). The filter cake was then dissolved in 48.3 litres of dimethylformamide at 55°C and the resulting solution diluted with about 180 litres of acetone. The resulting solution was filtered, the filtrate collected and about 11 litres of 28 percent aqueous ammonium hydroxide added very slowly to the filtrate maintained at about 35°C over a period of about ½ hour. During the addition of the aqueous ammonium hydroxide, the ammonium salt of 2-(4-chlorophenyl)-α-methyl-5-benzoxazolylacetic acid slowly precipitated yielding a slurry. After the addition of the ammonium hydroxide had been completed, the pH of the slurry was checked and found to be about 9. The slurry was next chilled in an ice-water mixture to about 0°C and the precipitated ammonium salt separated by filtration. The filter cake was washed with cold acetone (0°C) and the washed filter cake dried at 125°C for 3 hours in a tray dryer. During this heating and drying period, the ammonium salt decomposed yielding 2-(4-chlorophenyl)-α-methyl-5-benzoxazolylacetic acid as Form II. 29.65 kg of purified free acid were obtained assaying at about 95 percent purity. The presence of Form II was demonstrated using X-ray powder diffraction and infra red analysis. Using the same drying system, the following times and temperatures were found to give Form II (97% or higher purity) 6 hours at 95°C, 2.5 hours at 125°C, 1.5 hours at 140°C, 0.5 hours at 155°C, i.e. the higher the temperature used the faster will be the polymorphic transformation from Form I to Form II.

Example 2

Benoxaprofen (892 g) Form I was suspended in n-butyl acetate (9.8 litres) and the stirred suspension heated to the reflux temperature of the solvent to form a solution. The temperature of the solution was then slowly reduced (10°C every hour) until room temperature was reached.

The crystals of benoxaprofen thus produced were filtered off, washed with ethanol (892 ml) and dried *in vacuo* at 80°C. Yield 760 g.

The infra red spectrum and X-ray powder diffraction of the crystals showed that desired form II had been obtained.

Example 3

Tablets containing Benoxaprofen Form II were prepared using the following ingredients:

| Form II | Weight (mg) 100 |
| Starch | 55.1 |
| Polyvinylpyrrolidone | 8.25 |
| Magnesium Stearate | 1.65 |

The Form II and the starch were admixed and granulated with the polyvinylpyrrolidone as a 20% solution in water. Additional water was then added to form a suitable granulation which was passed through a stainless steel mesh screen with 1 mm apertures. The resultant granules were dried on a tray in a steam oven at 50 to 60°C. The dried granules were then passed through a screen (0.5 mm apertures) mixed with the magnesium stearate and compressed into tablets.

Tablets thus prepared were stored at 4, 25 and 40°C for two years. No deterioration in the physical characteristics of the tablets or in their appearance was noted over this period of time.

To demonstrate the metastable nature of benoxaprofen, the following test was run:

Benoxaprofen Form I was packed into glass ampoules (5 ml) and then subjected to cyclic temperature changes over two years. The weekly cycling programme adopted was that specified below:

| Monday | Tuesday | Wednesday | Thursday | Friday | Saturday | Sunday |
| --- | --- | --- | --- | --- | --- | --- |
| 37°C | 4°C | 15°C | 37°C | 4°C | 15°C | 15°C |

This test is designed to mimic actual storage conditions. After two years the benoxaprofen was analysed and it was found (by infra-red analysis) that no less than 20% by weight of Form I had undergone polymorphic transformation to Form II. This experiment clearly illustrates the metastable nature of Form I.

## Claims

1. Benoxaprofen Form II, benoxaprofen being 2-(4-chlorophenyl)-$\alpha$-methyl-5-benzoxazolylacetic acid, Form II crystals having the following powder diffraction pattern using filtered copper-nickel radiation at $\lambda = 1.5405$ Å.

| "d" in Å | $I/I_o$ |
| --- | --- |
| 11.77 | 10 |
| 8.06 | 10 |
| 7.07 | 70 |
| 5.67 | 100 |
| 5.30 | 10 |
| 5.06 | 20 |
| 4.79 | 10 |
| 4.41 | 50 |
| 4.17 | 80 |
| 3.93 | 05 |
| 3.65 | 30 |
| 3.56 | 90 |
| 3.24 | 60 |
| 3.09 | 40 |
| 3.03 | 15 |
| 2.97 | 15 |
| 2.81 | 05 |
| 2.75 | 05 |
| 2.66 | 05 |
| 2.57 | 05 |
| 2.37 | 10 |
| 2.29 | 05 |
| 2.15 | 05 |
| 2.04 | 15 |
| 1.98 | 20 |
| 1.91 | 05 |
| 1.78 | 02 |

2. Benoxaprofen Form II contaminated with less than 10% by weight of Form I; Form II being distinguishable from Form I by its infra red spectrum when homogeneously dispersed in a potassium bromide disc and employing a Perkin Elmer 297 Spectrophotometer, differences observed being as follows:

(a) Form I exhibits a sharp, medium intensity band at 880 cm$^{-1}$, whereas Form II exhibits a similar band at 885 cm$^{-1}$;

(b) in the region 1200—1330 cm$^{-1}$ both Forms show a similar positioning of bands, but the intensities differ, in Form I the bands at 1220 and 1250 cm$^{-1}$ being considerably more intense than the others, whilst in Form II all bands being of similar intensity; and

(c) a strong band near 1700 cm$^{-1}$ being considerably sharper for Form I than for Form II.

3. A pharmaceutical formulation of benoxaprofen associated with a pharmaceutically acceptable carrier therefor, characterised in that the benoxaprofen is benoxaprofen Form II, as defined in claim 1 or 2.

4. A pharmaceutical formulation according to claim 3 in the form of a tablet.

5. Benoxaprofen Form II, as defined in claim 1 or 2, for use as a pharmaceutical.

6. A method of preparing a pharmaceutical formulation of benoxaprofen, characterised in that benoxaprofen Form II, as defined in claim 1 or 2, is admixed with a pharmaceutical carrier therefor.

7. A method of preparing benoxaprofen Form II which comprises the slow and controlled crystallisation of benoxaprofen from a solution in n-butyl acetate.

8. A method of preparing benoxaprofen Form II which comprises thermally decomposing the ammonium salt of benoxaprofen at a temperature in the range 90—160°C.

## Revendications

1. Forme II de bénoxaprofen, le bénoxaprofen étant l'acide 2-(4-chlorophényl)-$\alpha$-

méthyl-5-benzoxazolylacétique, tandis que les cristaux de la forme II présentent le diagramme de diffraction de poudre ci-après en utilisant des radiations de cuivre-nickel filtrées à $\lambda=1,5405$ Å.

| "d" en Å | $I/I_o$ |
|---|---|
| 11,77 | 10 |
| 8,06 | 10 |
| 7,07 | 70 |
| 5,67 | 100 |
| 5,30 | 10 |
| 5,06 | 20 |
| 4,79 | 10 |
| 4,41 | 50 |
| 4,17 | 80 |
| 3,93 | 05 |
| 3,65 | 30 |
| 3,56 | 90 |
| 3,24 | 60 |
| 3,09 | 40 |
| 3,03 | 15 |
| 2,97 | 15 |
| 2,81 | 05 |
| 2,75 | 05 |
| 2,66 | 05 |
| 2,57 | 05 |
| 2,37 | 10 |
| 2,29 | 05 |
| 2,15 | 05 |
| 2,04 | 15 |
| 1,98 | 20 |
| 1,91 | 05 |
| 1,78 | 02 |

2. Forme II de bénoxaprofen contaminée avec moins de 10% en poids de la forme I; la forme II pouvant être distinguée de la forme I par son spectre d'absorption des rayons infrarouges lorsqu'elle est dispersée de manière homogène dans un disque de bromure de potassium et en employant un spectrophotomètre "Perkin Elmer 297", les différences observées étant les suivantes:

(a) la forme I présente une nette bande d'intensité moyenne à 880 cm$^{-1}$, tandis que la forme II présente une bande semblable à 885 cm$^{-1}$;

(b) dans la zone de 1.200—1.330 cm$^{-1}$, les deux formes présentent une localisation analogue de bandes, cependant que les intensités diffèrent, les bandes de la forme I à 1.220 et 1.250 cm$^{-1}$ étant beaucoup plus intenses que les autres tandis que, dans la forme II, toutes les bandes ont une intensité semblable; et

(c) une forte bande proche de 1.700 cm$^{-1}$ étant beaucoup plus nette pour la forme I que pour la forme II.

3. Formulation pharmaceutique de bénoxaprofen en association avec un support pharma-ceutiquement acceptable pour ce dernier, caractérisée en ce que le bénoxaprofen est la forme II de bénoxaprofen selon les définitions données dans la revendication 1 ou 2.

4. Formulation pharmaceutique suivant la revendication 3, sous forme d'un comprimé.

5. Forme II de bénoxaprofen selon les définitions données dans la revendication 1 ou 2, utilisée comme produit pharmaceutique.

6. Procédé de préparation d'une formulation pharmaceutique de bénoxaprofen, caractérisé en ce qu'on mélange la forme II de bénoxa-profen selon les définitions données dans la revendication 1 ou 2, avec un support pharmaceutique pour cette dernière.

7. Procédé de préparation de la forme II de bénoxaprofen, caractérisé en ce qu'il consiste à effectuer la cristallisation lente et contrôlée du bénoxaprofen hors d'une solution dans de l'acétate de n-butyle.

8. Procédé de préparation de la forme II de bénoxaprofen, caractérisé en ce qu'il consiste à décomposer thermiquement le sel d'ammonium de bénoxaprofen à une température se situant dans l'intervalle de 90—160°C.

**Patentansprüche**

1. Benoxaprofen Form II, wobei Benoxa-profen 2-(4-Chlorphenyl)-$\alpha$-methyl-5-benzoxa-zolylessigsäure ist, und wobei die Form II Kristalle das folgende Pulver-Beugungsbild bei Verwendung von gefilterter Kupfer-Nickel-Strahlung bei $\lambda=1.5405$ Å ergeben:

| "d" in Å | $I/I_o$ |
|---|---|
| 11.77 | 10 |
| 8.06 | 10 |
| 7.07 | 70 |
| 5.67 | 100 |
| 5.30 | 10 |
| 5.06 | 20 |
| 4.79 | 10 |
| 4.41 | 50 |
| 4.17 | 80 |
| 3.93 | 05 |
| 3.65 | 30 |
| 3.56 | 90 |
| 3.24 | 60 |
| 3.09 | 40 |
| 3.03 | 15 |
| 2.97 | 15 |
| 2.81 | 05 |
| 2.75 | 05 |
| 2.66 | 05 |
| 2.57 | 05 |
| 2.37 | 10 |
| 2.29 | 05 |
| 2.15 | 05 |
| 2.04 | 15 |
| 1.98 | 20 |
| 1.91 | 05 |
| 1.78 | 02 |

2. Benoxaprofen Form II, verunreinigt durch weniger als 10 Gew.-% der Form I, wobei Form II von der Form I unterscheidbar ist durch das Infrarotspektrum bei homogener Verteilung in einer Kaliumbromid-Scheibe und bei Ver-

wendung eines Perkin Elmer 297 Spectrophotometer, wobei die folgenden Unterschiede beobachtet werden:

(a) Form I ergibt ein scharfes Band bei 880 $cm^{-1}$ von mittlerer Intensität, während Form II ein ähnliches Band bei 885 $cm^{-1}$ ergibt;

(b) im Bereich 1200 bis 1330 $cm^{-1}$ zeigen beide Formen eine ähnliche Anordnung von Banden, aber die Intensitäten sind dahingehend unterschiedlich, daß in Form I die Banden bei 1220 und 1250 $cm^{-1}$ beträchtlich intensiver sind als die anderen, während in Form II alle Banden von ähnlicher Intensität sind; und

(c) eine starke Bande nahe 1700 $cm^{-1}$ ist beträchtlich schärfer bei Form I als bei Form II.

3. Pharmazeutische Zubereitung enthaltend Benoxaprofen zusammen mit einem pharmazeutisch annehmbaren Träger dafür, dadurch gekennzeichnet, daß das Benoxaprofen das Benoxaprofen Form II gemäß Anspruch 1 oder 2 ist.

4. Pharmazeutische Zubereitung gemäß Anspruch 3 in Form von Tabletten.

5. Verwendung von Benoxaprofen Form II gemäß Anspruch 1 oder 2 als Pharmazeutikum.

6. Verfahren zur Herstellung einer pharmazeutischen Zubereitung von Benoxaprofen, dadurch gekennzeichnet, daß Benoxaprofen Form II gemäß Anspruch 1 oder 2 mit einem pharmazeutischen Träger dafür vermischt wird.

7. Verfahren zur Herstellung von Benoxaprofen Form II, gekennzeichnet durch eine langsame und kontrollierte Kristallisation von Benoxaprofen aus einer Lösung in n-Butylacetat.

8. Verfahren zur Herstellung von Benoxaprofen Form II, gekennzeichnet, durch eine thermische Zersetzung des Ammoniumsalzes von Benoxaprofen bei einer Temperatur im Bereich von 90 bis 160°C.